(19) Europäisches Patentamt
European Patent Office
Office européen des brevets

(11) **EP 2 196 136 A1**

(12) **DEMANDE DE BREVET EUROPEEN**

(43) Date de publication:
**16.06.2010 Bulletin 2010/24**

(51) Int Cl.:
**A61B 5/00** *(2006.01)*    **A61B 5/15** *(2006.01)*

(21) Numéro de dépôt: **09290874.8**

(22) Date de dépôt: **20.11.2009**

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **11.12.2008  FR 0806969**

(71) Demandeur: **Etat Français représenté par le Délégué Général pour L'Armement 94114 Arcueil Cedex (FR)**

(72) Inventeurs:
• **Le Breton-Guinet, Angélique 38190 LAVAL (FR)**
• **Besnard, Yves 38400 SAINT-MARTIN D'HERES (FR)**
• **Launay, Jean-Claude 38000 GRENOBLE (FR)**
• **Savourey, Gustave 38330 BIVERS (FR)**

(54) **Procédé de détermination de la quantité Cp02 (to) d'oxygène 02 contenu dans le sang et dispositif associé**

(57)    L'invention concerne le domaine des dispositifs ou procédés de diagnostic, et a pour objet un procédé de détermination des variations du contenu en oxygène du sang périphérique comportant les étapes suivantes :
- Détermination simultanée de la concentration en hémoglobine et du taux de saturation d'oxygène dans le sang à un instant $t_0$ ;
- Calcul de la quantité d'oxygène contenue dans le sang périphérique puis mise en mémoire de cette valeur,

ainsi qu'un dispositif associé comportant
- des premiers moyens 2 de mesure de la concentration en hémoglobine contenue dans le sang périphérique,
- des seconds moyens 3 de mesure du taux de saturation d'oxygène du sang périphérique,
- des moyens 4 de commande du déclenchement simultané des premiers et seconds moyens de mesure
- des moyens 5 de calcul de la quantité d'oxygène contenue dans le sang périphérique,
- des moyens 6 de mise en mémoire d'informations.

Fig. 1

EP 2 196 136 A1

**Description**

Le domaine technique de l'invention est celui des instruments, dispositifs ou procédés de mesures physiologiques _et a plus particulièrement pour objet un procédé de détermination indirecte de variations du contenu en oxygène du sang périphérique ainsi qu'un dispositif associé nommé oxycontentomètre.

Le brevet DE102006051561 décrit un dispositif général faisant appel à des capteurs electromagnétiques, optiques, acoustiques pour des mesures physiologiques diverses non décrites. Il décrit plus particulièrement un procédé de détermination de $CaO_2$,c'est-à-dire du contenu artériel en $O_2$ qui nécessite pour sa détermination un prélèvement sanguin ARTERIEL sur lequel on mesure la saturation de l'Hb en $O_2$ ($SaO_2$) d'une part et la concentration de l'hémoglobine du sang ARTERIEL (cHb) d'autre part. Ce procédé présente deux inconvéneints majeurs, à savoir d'une part que ce prélèvement artériel nécessite une ponction d'une artère ne pouvant être réalisée que par un médecin et que d'autre part, il n'existe pas, à ce jour, de possibilité de mesure de Ca02 de façon indirecte par un capteur placé sur un doigt ou ailleurs.

La problématique de détermination indirecte et non invasive du contenu en oxygène du sang périphérique connaît déjà plusieurs dispositifs notamment celui des oxymètres à photométrie spectrale. A ce titre, on connaît le brevet US6622095 qui décrit un dispositif de détermination de concentrations en hémoglobine **caractérisé en ce qu'**il comporte trois sources émettrices de faisceaux lumineux de longueurs d'onde respectivement rouge, proche de l'infrarouge et rouge orangé. Plus particulièrement, ces faisceaux sont émis ou reflétés au travers d'un tissu vivant au moment d'une onde pulsatile. Ledit dispositif est alors **caractérisé en ce qu'**il est apte à recueillir le signal lumineux et la différence d'absorption permet de déterminer la saturation de l'hémoglobine en oxygène en pourcentage et ainsi les variations de concentration en oxygène.

Un tel dispositif a notamment l'inconvénient d'indiquer des valeurs en pourcentages via un taux de saturation de l'hémoglobine en oxygène. De plus ce dispositif n'indique qu'un taux à un instant $t$ et ne permet pas de suivre l'évolution de la quantité d'hémoglobine et n'est pas apte à être utilisé dans le cadre de dépistages de prévention ou de suivis rigoureux des troubles hypoxémiques.

Par ailleurs, le document intitulé « Normo or hypobaric hypoxic tests : propositions for the determination of the individual susceptibility to altitude illness" G. Savourey et Al. european journal of applied physiology, Springer Berlin, DE, Vol. 100, n° 2, 24 février 2007 pp. 193-205 décrit une relation entre $CpO_2$ à cHb et $SpO_2$ et indique que la la variable $CpO_2$ est une variable importante pour prédire le score maximal au questionnaire de Lake Louise utilisé pour la quantification du mal aigu des montagnes. et il les algorithmes de détermination des risques d'apparition de mal aigu des montagnes et de mal adaptation montre que la variable $CpO_2$ est nécessaire mais non suffisante pour ces déterminations.

L'un des buts de l'invention est de résoudre cet inconvénient en proposant un dispositif apte à indiquer des quantités d'oxygène dans le sang en valeur absolue, de façon indirecte et permettant également de suivre l'évolution de ces données pour en indiquer les variations de manière quantifiée.

La solution apportée est un procédé de détermination de la quantité $CpO_2(t_0)$ d'oxygène $O_2$ contenu dans le sang périphérique et éventuellement de la variation de quantité d'oxygène contenue dans le sang , mis en oeuvre par un dispositif comportant des moyens de mesures, des moyens de calculs, des moyens de comptage horaire et des moyens de mise en mémoire et, préférablement, des moyens d'affichage et/ou des moyens de génération d'un signal d'alarme, ce procédé comportant, selon une première caractéristique, les étapes suivantes :

-   Détermination simultanée de la concentration en hémoglobine [$Hb$] et du taux de saturation d'oxygène dans le sang $SpO_2$ à un instant $t_0$ ;
-   Calcul de la quantité $CpO_2(t_0)$ d'oxygène $O_2$ contenu dans le sang périphérique à l'instant $t_0$ à partir de la concentration en hémoglobine [$Hb$] et du taux de saturation d'oxygène dans le sang $SpO_2$, à l'instant $t_0$;
-   Mise en mémoire de ladite valeur de $CpO_2(t_0)$ calculée précédemment.

Un procédé selon l'invention peut permettre de constater l'évolution du $CpO_2$, la personne ayant mis en oeuvre ce procédé pouvant alors évaluer l'évolution de son Cp02 par extrapolation et éventuellement prévenir des risques de troubles hypoxémiques.

En aucun cas ce procédé ne permet de traiter ni médicalement et encore moins chirurgicalement le corps humain ou animal dans le sens où le procédé n'a pas pour effet de prévenir ou de traiter un état pathologique et ne possède donc pas d'effets thérapeutiques . En outre, la détermination de $CpO_2$ ne constitue pas un diagnostic car cette détermination ne permet pas à elle seule de prendre directement une décision au sujet d'un traitement médical et tout particulièrement pour traiter une dysoxie.

Selon une caractéristique particulière, un procédé selon l'invention comporte, en outre, les étapes suivantes :

-   Détermination simultanée de la concentration en hémoglobine [$Hb$] et du taux de saturation d'oxygène dans le sang $SpO_2$ à un instant ($t_0$+$T$) ;

- Calcul de la quantité $CpO_2(t_0+T)$ d'oxygène $O_2$ contenue dans le sang périphérique à l'instant $(t_0+T)$ à partir de la concentration en hémoglobine $[Hb]$ et du taux de saturation d'oxygène dans le sang $SpO_2$, à l'instant $(t_0+T)$;
- Mise en mémoire de ladite valeur de $CpO_2(t_0+T)$ déterminée précédemment;
- Calcul de la variation de quantité d'oxygène contenue dans le sang périphérique entre les instants $t_0$ et $(t_0+T)$ :

$$\Delta CpO_2\left(\!\left[t_0;t_0+T\right]\!\right) = CpO_2(t_0+T) - CpO_2(t_0) \ ;$$

- Mise en mémoire de ladite valeur $\Delta CpO_2([t_0;t_0+T])$ et, préférentiellement, affichage de ladite valeur $\Delta CpO_2(t_0+T)$ calculée précédemment et/ou génération d'un signal d'alarme si cette valeur dépasse un seuil prédéterminé.

Selon une caractéristique particulière, un procédé selon l'invention comporte les étapes suivantes :

- Détermination simultanée de la concentration en hémoglobine $[Hb]$ exprimée en $g.dL^{-1}$ et du taux de saturation d'oxygène dans le sang $SpO_2$ exprimé en pourcentage à un instant $t_0$ ;
- Calcul de la quantité $CpO_2(t_0)$ d'oxygène $O_2$ contenue dans le sang périphérique exprimée en $mL.dL^{-1}$ ;
- Mise en mémoire de ladite valeur de $CpO_2(t_0)$ déterminée précédemment ;
- Détermination simultanée de la concentration en hémoglobine $[Hb]$ exprimée en $g.dL^{-1}$ et du taux de saturation d'oxygène dans le sang $SpO_2$ exprimé en pourcentage à un instant $(t_0+T)$ ;
- Calcul de la quantité $CpO_2(t_0+T)$ d'oxygène $O_2$ contenue dans le sang périphérique exprimée en $mL.dL^{-1}$ ;
- Mise en mémoire de ladite valeur de $CpO_2(t_0+T)$ déterminée précédemment;
- Calcul de la variation de quantité d'oxygène contenu dans le sang périphérique entre les instants $t_0$ et $(t_0+T)$ :

$$\Delta CpO_2\left(\!\left[t_0;t_0+T\right]\!\right) = CpO_2(t_0+T) - CpO_2(t_0) \ ;$$

- et, préférablement, affichage de ladite valeur $\Delta CpO_2(t_0+T)$ calculée précédemment.

Selon une caractéristique additionnelle, un procédé selon l'invention comporte une étape de calcul de la quantité $CpO_2(t_0+T)$ d'oxygène $O_2$ contenu dans le sang périphérique exprimée en $mL.dL^{-1}$ à partir de la formule suivante :

$$CpO_2 = 1{,}34.[Hb].SpO_2$$

où l'on retrouve le terme de la concentration en hémoglobine $[Hb]$ exprimée en $g.dL^{-1}$ et le terme du taux de saturation d'oxygène dans le sang $SpO_2$ exprimé en pourcentage à un certain instant $t$.

Le coefficient *1,34* correspond au pouvoir oxyphorique moyen de l'hémoglobine humaine exprimé en $mL.g^{-1}$. Une variation de ladite quantité $CpO_2$ d'oxygène $O_2$ contenu dans le sang périphérique entre deux instants est donc définie par la différence entre les deux valeurs obtenues aux instants respectifs.

Selon une caractéristique particulière, l'étape de détermination de la concentration en hémoglobine $[Hb]$ comporte les étapes suivantes :

- Microponction capillaire d'un volume déterminé $V_{dét}$ de sang;
- Analyse photométrique dudit sang prélevé;

Selon une autre caractéristique particulière, l'étape de détermination du taux de saturation d'oxygène dans le sang $SpO_2$ exprimé en pourcentage est déterminé par un capteur oxymétrique.

L'invention concerne aussi un dispositif, nommé oxycontentomètre, de détermination de la quantité $CpO_2(t_0)$ d'oxygène $O_2$ contenu dans le sang périphérique et éventuellement de la variation de quantité d'oxygène contenu dans le sang périphérique **caractérisé en ce qu'**il comporte :

- des premiers moyens de mesure de la concentration en hémoglobine $[Hb]$ contenue dans le sang périphérique,
- des seconds moyens de mesure du taux de saturation d'oxygène $SpO_2$ du sang périphérique,
- des moyens de commande du déclenchement simultané des premiers et seconds moyens de mesure
- des moyens de calcul aptes à calculer la quantité $CpO_2(t_0)$ d'oxygène $O_2$ contenue dans le sang périphérique

- des moyens de mise en mémoire d'informations.

Selon une caractéristique particulière, un dispositif selon l'invention comporte au moins l'une des caractéristiques suivantes :

- - des moyens de comptage horaire aptes à mesurer le temps écoulé entre deux mesures successives et des moyens de calcul aptes à calculer la variation de quantité d'oxygène contenu dans le sang périphérique entre les instants $t_0$ et $(t_0+T)$ :

$$\Delta CpO_2\left(\left[t_0 ; t_0 + T\right]\right) = CpO_2(t_0 + T) - CpO_2(t_0)$$

- un capteur oxymétrique ;
- un dispositif de microponctions capillaires ;
- un oxymètre de pouls couplé au dit dispositif de microponctions capillaires.
- les moyens de commande du déclenchement simultané des premiers et seconds moyens de mesure sont constitués par un micro interrupteur.

D'autres avantages et caractéristiques apparaîtront dans la description d'un mode de réalisation de l'invention au regard des figures annexées parmi lesquelles :

- la figure 1 montre un schéma d'un dispositif de détermination de variations d'oxygène dans le sang selon un mode de réalisation de l'invention,
- la figure 2 présente un exemple de réalisation des premiers moyens 2 de mesure de la concentration en hémoglobine [*Hb*] contenue dans le sang périphérique,
- la figure 3 montre un exemple d'agencement des moyens constitutifs d'un dispositif selon la figure 1,
- la figure 4 montre plus en détail un schéma des premiers moyens de mesure de la concentration en hémoglobine [*Hb*] contenue dans le sang périphérique.

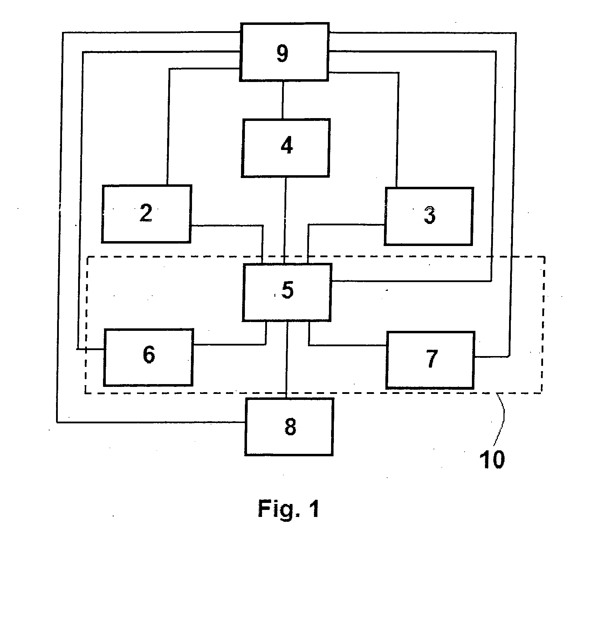

La figure 1 représente un schéma d'un dispositif de détermination de variations d'oxygène dans le sang selon un mode de réalisation de l'invention. Ce dispositif comporte :

- des premiers moyens 2 de mesure de la concentration en hémoglobine [*Hb*] contenue dans le sang périphérique,
- des seconds moyens 3 de mesure du taux de saturation d'oxygène $SpO_2$ du sang périphérique,
- des moyens 4 de commande du déclenchement des premiers et seconds moyens de mesure
- des moyens 5 de calcul de la quantité $CpO_2(t_0)$ d'oxygène $O_2$ contenue dans le sang périphérique
- des moyens 6 de mise en mémoire d'informations
- des moyens 7 de comptage horaire aptes à mesurer le temps écoulé entre deux mesures successives.
- des moyens 8 d'affichage d'information et ou de génération d'un signal d'alarme.
- des moyens d'alimentation électrique 9 des moyens précités.

Dans ce mode de réalisation particulier, les moyens 5 de calcul de la quantité $CpO_2(t_0)$ d'oxygène $O_2$ contenue dans le sang périphérique, les moyens 6 de mise en mémoire d'informations et les moyens 7 de comptage horaire sont constitués par un microprocesseur 10 et ses périphériques.

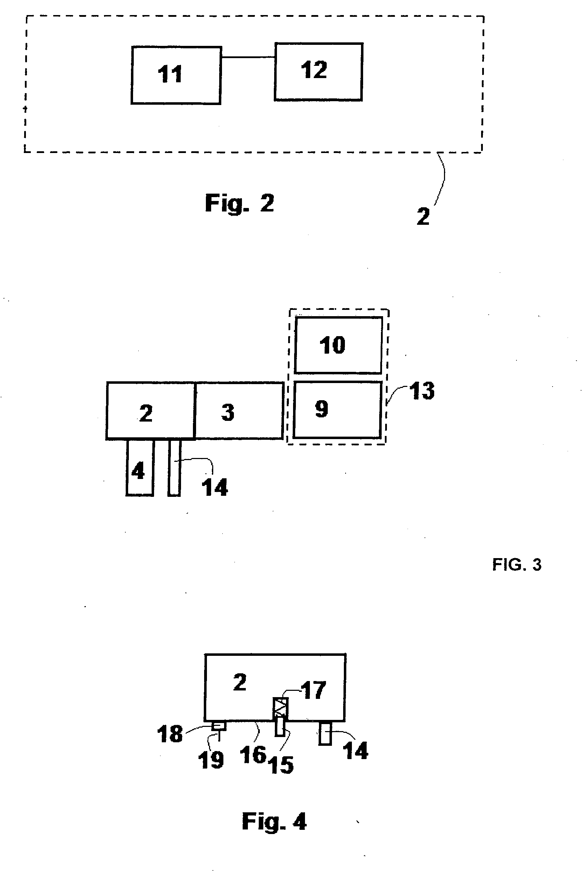

Les premiers moyens 2 de mesure de la concentration en hémoglobine [*Hb*] contenue dans le sang périphérique sont constitués, de façon connue et comme montré sur la figure 2 par des moyens 11 de microponction capillaire 10 d'un volume déterminé $V_{dét}$ de sang et des moyens 12 d'analyse photométrique 11 dudit sang prélevé tandis que les seconds moyens 3 de mesure du taux de saturation d'oxygène $SpO_2$ du sang périphérique sont constitués par un capteur oxymétrique, tel par exemple un oxymètre de pouls.

Les moyens 4 de commande du déclenchement des premiers et seconds moyens de mesure peuvent être manuels et par exemple être constitués par un interrupteur déclenchable manuellement par l'utilisateur du dispositif ou automatique et être préprogrammé au sein du microprocesseur 10 de façon à être déclenché régulièrement ou non, l'intervalle entre deux mesures pouvant par exemple être d'autant plus faible que la valeur calculée du $CpO_2(t_0)$ d'oxygène $O_2$ contenue dans le sang périphérique est proche d'une valeur seuil. Comme montré sur la figure 3, le microprocesseur 10 et l'alimentation électrique 9 sont intégrés sur une carte à puce 13 tandis que les moyens de commande 4 sont associés aux premiers moyens 2 de mesure de la concentration en hémoglobine [*Hb*] contenue dans le sang périphérique.

Ces premiers moyens 2, , les seconds moyens 3 de mesure du taux de saturation d'oxygène $SpO_2$ du sang périphérique et la carte à puce 13 sont agencés de façon contiguë et solidaire d'une bague en matériau élastique 14.

La figure 4 montre plus précisément un exemple d'un schéma d'agencement des moyens constitutifs de ce mode de réalisation de l'invention.

Les moyens de commande 4 sont constitués par un micro interrupteur 15 de type bouton poussoir. Ce micro interrupteur est rétractable à l'intérieur d'une cavité pratiquée sur la surface inférieure 16 des premiers moyens 2 de mesure de la concentration en hémoglobine [Hb] contenue dans le sang périphérique. Il est en outre maintenu en position sortie par un ressort 17. Cette surface inférieure 16 comporte aussi un embout amovible 18 comportant une micro aiguille 19 disposée parallèlement à l'interrupteur et de longueur sensiblement égale à celle de la partie du micro interrupteur émergeant de ladite face inférieure. Une bague élastique 14 est aussi fixée sur ladite face inférieure 16 et de sorte que l'interrupteur soit positionné entre ladite aiguille 17 et ladite bague 14. Par ailleurs, l'aiguille est protégée par un capuchon non représenté avant son utilisation

Le fonctionnement de ce dispositif est le suivant :

Le dispositif est fixé sur l'un des doigts de la main de l'utilisateur, par exemple le majeur, à l'aide de la bague élastique 14 et de sorte que l'interrupteur soit en contact avec la peau du doigt. Le capuchon de protection de l'aiguille est ensuite ôté et les mesures peuvent ensuite être effectuées. Une poussée, en direction du doigt concerné, sur les premiers moyens 2 de mesure de la concentration en hémoglobine [Hb] provoque la rétraction du micro interrupteur à l'intérieur de la cavité et son actionnement. Dans cette position, d'une part l'aiguille stérile pénètre dans la peau de l'utilisateur et prélève une petite quantité de sang et, d'autre part, les mesures, au temps to généré par l'actionnement du micro interrupteur, de la concentration en hémoglobine [Hb] et du taux de saturation d'oxygène $SpO_2$ sont lancées simultanément via le microprocesseur qui génère une impulsion de commande du fonctionnement des premiers moyens 2 de mesure de la concentration en hémoglobine [Hb] contenue dans le sang périphérique, les seconds moyens 3 de mesure du taux de saturation d'oxygène $SpO_2$ du sang périphérique. Les moyens de calcul calculent ensuite la quantité $CpO_2(t_0)$ d'oxygène $O_2$ contenue dans le sang périphérique à partir desdites valeurs mesurées par les premiers et seconds moyens de mesure en utilisant la formule suivante :

$$CpO_2 = 1,34.[Hb].SpO_2$$

où l'on retrouve le terme de la concentration en hémoglobine [Hb] exprimée en $g.dL^{-1}$ et le terme du taux de saturation d'oxygène dans le sang $SpO_2$ exprimé en pourcentage à un certain instant to. Cette valeur calculée $CpO_2(t_0)$ est ensuite mise en mémoire dans les moyens de mise en mémoire. Par ailleurs, le microprocesseur déclenche les moyens de comptage horaire.

L'embout 18 utilisé est ensuite remplacé par un autre embout afin de changer de microaiguille et de pouvoir ainsi renouveler les étapes précédemment mentionnées et de calculer la quantité $CpO_2(t_0)$ d'oxygène $O_2$ contenue dans le sang périphérique à l'instant to+ T.

Les moyens de calcul sont alors aptes à calculer la variation de quantité d'oxygène contenu dans le sang périphérique entre les instants $t_0$ et $(t_0+T)$ :

$$\Delta CpO_2([t_0;t_0+T]) = CpO_2(t_0+T) - CpO_2(t_0) \; ;$$

et à afficher ladite valeur $\Delta CpO_2(t_0+T)$ calculée précédemment et/ou à générer un signal d'alarme si cette valeur dépasse un seuil prédéterminé.

Un procédé selon l'invention permet donc de mesurer la quantité d'oxygène contenu dans le sang périphérique et ses variations dans le temps. Ces mesures physiologiques peuvent par exemple constituer des mesures intermédiaires pour le diagnostic du mal aigu des montagnes. En effet, ces mesures seront prises en compte par le médecin avec l'ensemble des autres données telles que l'anamnèse, l'examen clinique et para clinique..., pour établir le diagnostic.

De nombreuses modifications peuvent être apportées au mode de réalisation décrit précédemment sans sortir du cadre de l'invention. Ainsi, les moyens de commande du déclenchement simultané des premiers et seconds moyens de mesure peuvent par exemple être associé directement à l'aiguille.

**Revendications**

1. Procédé de détermination de la quantité $CpO_2(t_0)$ d'oxygène $O_2$ contenu dans le sang périphérique et éventuellement de la variation de quantité d'oxygène contenu dans le sang comportant des moyens de mesures, des moyens de calculs, des moyens de comptage horaire et des moyens de mise en mémoire et, préférablement, des moyens d'affichage et/ou des moyens de génération d'un signal d'alarme, **caractérisé en ce qu'**il comporte les étapes suivantes :

   - Détermination simultanée de la concentration en hémoglobine [$Hb$] et du taux de saturation d'oxygène dans le sang $SpO_2$ à un instant $t_0$ ;
   - Calcul de la quantité $CpO_2(t_0)$ d'oxygène $O_2$ contenu dans le sang périphérique à l'instant $t_0$ à partir de la concentration en hémoglobine [$Hb$] et du taux de saturation d'oxygène dans le sang $SpO_2$, à l'instant $t_0$
   - Mise en mémoire de ladite valeur de $CpO_2(t_0)$ calculée précédemment.

2. Procédé selon la revendication 1, **caractérisé en ce qu'**il comporte en outre les étapes suivantes :

   - Détermination simultanée de la concentration en hémoglobine [$Hb$] et du taux de saturation d'oxygène dans le sang $SpO_2$ à un instant ($t_0+T$) ;
   - Calcul de la quantité $CpO_2(t_0+T)$ d'oxygène $O_2$ contenue dans le sang périphérique à l'instant ($t_0+T$) à partir de la concentration en hémoglobine [$Hb$] et du taux de saturation d'oxygène dans le sang $SpO_2$, à l'instant ($t_0+T$);
   - Mise en mémoire de ladite valeur de $CpO_2(t_0+T)$ déterminée précédemment;
   - Calcul de la variation de quantité d'oxygène contenue dans le sang périphérique entre les instants $t_0$ et ($t_0+T$) :

$$\Delta CpO_2\big([t_0;t_0+T]\big) = CpO_2(t_0+T) - CpO_2(t_0) \ ;$$

   - Mise en mémoire de ladite valeur $\Delta CpO_2([t_0;t_0+T])$ et, préférentiellement, affichage de ladite valeur $\Delta CpO_2$ ($t_0+T$) calculée précédemment et/ou génération d'un signal d'alarme si cette valeur dépasse un seuil prédéterminé.

3. Procédé selon l'une quelconque des revendications 1 et 2, **caractérisé en ce qu'**il comporte en outre les étapes suivantes

   - Détermination simultanée de la concentration en hémoglobine [$Hb$] exprimée en $g.dL^{-1}$ et du taux de saturation d'oxygène dans le sang $SpO_2$ exprimé en pourcentage à un instant $t_0$ ;
   - Calcul de la quantité $CpO_2(t_0)$ d'oxygène $O_2$ contenue dans le sang périphérique exprimée en $mL.dL^{-1}$ ;
   - Mise en mémoire de ladite valeur de $CpO_2(t_0)$ déterminée précédemment
   - Détermination simultanée de la concentration en hémoglobine [$Hb$] exprimée en $g.dL^{-1}$ et du taux de saturation d'oxygène dans le sang $SpO_2$ exprimé en pourcentage à un instant ($t_0+T$)
   - Calcul de la quantité $CpO_2(t_0+T)$ d'oxygène $O_2$ contenue dans le sang périphérique exprimée en $mL.dL^{-1}$
   - Mise en mémoire de ladite valeur de $CpO_2(t_0+T)$ déterminée précédemment
   - Calcul de la variation de quantité d'oxygène contenu dans le sang périphérique entre les instants $t_0$ et ($t_0+T$) :

$$\Delta CpO_2\big([t_0;t_0+T]\big) = CpO_2(t_0+T) - CpO_2(t_0)$$

4. Procédé selon la revendication 1, **caractérisé en ce qu'**il comporte une étape de calcul la quantité $CpO_2(t_0+T)$ d'oxygène $O_2$ contenu dans le sang périphérique exprimée en $mL.dL^{-1}$ à partir de la formule suivante :

$$CpO_2 = 1,34.[Hb].SpO_2$$

où l'on retrouve le terme de la concentration en hémoglobine [$Hb$] exprimée en $g.dL^{-1}$ et le terme du taux de saturation d'oxygène dans le sang $SpO_2$ exprimé en pourcentage à un certain instant $t$.

**5.** ; Procédé selon l'une quelconque des revendications 1 à 4, **caractérisé en ce que** l'étape de détermination de la concentration en hémoglobine [*Hb*] comporte les étapes suivantes :

- Microponction capillaire d'un volume déterminé $V_{dét}$ de sang;
- Analyse photométrique dudit sang prélevé;

**6.** Procédé selon l'une quelconque des revendications 1 à 5, , **caractérisé en ce que** l'étape de détermination du taux de saturation d'oxygène dans le sang $SpO_2$ exprimé en pourcentage est déterminé par un capteur oxymétrique.

**7.** Dispositif de détermination de la quantité $CpO_2(t_0)$ d'oxygène $O_2$ contenu dans le sang périphérique et éventuellement de la variation de quantité d'oxygène contenu dans le sang périphérique **caractérisé en ce qu'**il comporte :

- des premiers moyens 2 de mesure de la concentration en hémoglobine [*Hb*] contenue dans le sang périphérique,
- des seconds moyens 3 de mesure du taux de saturation d'oxygène $SpO_2$ du sang périphérique,
- des moyens 4 de commande du déclenchement simultané des premiers et seconds moyens de mesure
- des moyens 5 de calcul aptes à calculer la quantité $CpO_2(t_0)$ d'oxygène $O_2$ contenue dans le sang périphérique
- des moyens 6 de mise en mémoire d'informations.

**8.** Dispositif selon la revendication 7, **caractérisé en ce qu'**il comporte en outre des moyens 7 de comptage horaire aptes à mesurer le temps écoulé entre deux mesures successives et des moyens de calcul aptes à calculer la variation de quantité d'oxygène contenu dans le sang périphérique entre les instants $t_0$ et $(t_0+T)$ :

$$\Delta CpO_2\left(\left[t_0; t_0 + T\right]\right) = CpO_2(t_0 + T) - CpO_2(t_0)$$

**9.** Dispositif selon l'une quelconque des revendications 7 et 8, **caractérisé en ce qu'**il comporte

- Un capteur oxymétrique ;
- Un dispositif de microponctions capillaires ;
- Un oxymètre de pouls couplé au dit dispositif de microponctions capillaires.

**10.** Dispositif selon l'une quelconque des revendications 7 à 9, **caractérisé en ce que** les moyens 4 de commande du déclenchement simultané des premiers et seconds moyens de mesure sont constitués par un micro interrupteur.

Fig. 1

**Fig. 2**

2

**FIG. 3**

**Fig. 4**

Europäisches
Patentamt

European
Patent Office

Office européen
des brevets

**RAPPORT PARTIEL
DE RECHERCHE EUROPEENNE**
qui selon la règle 63 de la Convention sur le brevet
européen est consideré, aux fins de la procédure ultérieure,
comme le rapport de la recherche européenne

Numéro de la demande

EP 09 29 0874

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | DE 10 2006 051561 A1 (WEINMANN G GERAETE MED [DE]) 24 mai 2007 (2007-05-24) * alinéas [0015] - [0027] * * alinéas [0044] - [0054] * * alinéa [0080] * ----- | 7-8,10 | INV. A61B5/00 A61B5/15 |
| Y | GUSTAVE SAVOUREY ET AL: "Normo or hypobaric hypoxic tests: propositions for the determination of the individual susceptibility to altitude illnesses" EUROPEAN JOURNAL OF APPLIED PHYSIOLOGY, SPRINGER, BERLIN, DE, vol. 100, no. 2, 24 février 2007 (2007-02-24), pages 193-205, XP019517306 ISSN: 1439-6327 * Calculations p. 197 * * Discussion p. 201-204 * ----- | 7-10 | |
| Y | US 2007/110621 A1 (MACINTYRE DUNCAN [CA] ET AL) 17 mai 2007 (2007-05-17) * alinéas [0025], [0026] * * alinéas [0050] - [0054] * * alinéas [0062] - [0071] * ----- | 7-10 | DOMAINES TECHNIQUES RECHERCHES (IPC) A61B |

-/--

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| Berlin | 1 mars 2010 | Trachterna, Morten |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.................................................................
& : membre de la même famille, document correspondant

EPO FORM 1503 03.82 (P04E08)

Europäisches
Patentamt

European
Patent Office

Office européen
des brevets

**RAPPORT PARTIEL
DE RECHERCHE EUROPEENNE**

Numero de la demande

EP 09 29 0874

| | DOCUMENTS CONSIDERES COMME PERTINENTS | | CLASSEMENT DE LA DEMANDE (IPC) |
|---|---|---|---|
| Catégorie | Citation du document avec indication, en cas de besoin, des parties pertinentes | Revendication concernée | |
| X | US 5 101 825 A (GRAVENSTEIN DIETRICH [US] ET AL) 7 avril 1992 (1992-04-07) * colonne 2, ligne 63 - colonne 4, ligne 59 * ----- | 7-8,10 | |
| | | | **DOMAINES TECHNIQUES RECHERCHES (IPC)** |

EPO FORM 1503 03.82 (P04C11)

Europäisches
Patentamt

European
Patent Office

Office européen
des brevets

**RECHERCHE INCOMPLETE**
**FEUILLE SUPPLEMENTAIRE C**

Numéro de la demande

EP 09 29 0874

Revendications susceptibles de faire l'objet de recherches complètes:
7-10

Revendications n'ayant pas fait l'objet de recherches:
1-6

Raison pour la limitation de la recherche (invention(s) non brevetable(s)):

Méthode de traitement chirurgical du corps humain ou animal

Les revendications 1-6 se réfèrent à une méthode de traitement chirurgical du corps humain ou animal, en ce qui concerne l'étape de la détermination de la concentration en hémoglobine par microponction capillaire.

**EP 2 196 136 A1**

## ANNEXE AU RAPPORT DE RECHERCHE EUROPEENNE
## RELATIF A LA DEMANDE DE BREVET EUROPEEN NO.

EP 09 29 0874

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

01-03-2010

| Document brevet cité au rapport de recherche | Date de publication | Membre(s) de la famille de brevet(s) | | Date de publication |
|---|---|---|---|---|
| DE 102006051561 A1 | 24-05-2007 | AUCUN | | |
| US 2007110621 A1 | 17-05-2007 | WO | 2007028231 A1 | 15-03-2007 |
| | | EP | 1928314 A1 | 11-06-2008 |
| | | US | 2009221886 A1 | 03-09-2009 |
| US 5101825 A | 07-04-1992 | AT | 146952 T | 15-01-1997 |
| | | AU | 4636089 A | 14-05-1990 |
| | | CA | 2001455 A1 | 28-04-1990 |
| | | DE | 68927614 D1 | 13-02-1997 |
| | | DE | 68927614 T2 | 28-05-1997 |
| | | EP | 0440745 A1 | 14-08-1991 |
| | | WO | 9004353 A2 | 03-05-1990 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- DE 102006051561

- US 6622095 B

**Littérature non-brevet citée dans la description**

- Normo or hypobaric hypoxic tests : propositions for the determination of the individual susceptibility to altitude illness. **G. Savourey et al.** european journal of applied physiology. Springer Berlin, 24 Février 2007, vol. 100, 193-205